(19) Europäisches Patentamt
European Patent Office
Office européen des brevets

(11) Numéro de publication: **0 053 981 B1**

# (12) FASCICULE DE BREVET EUROPEEN

(45) Date de publication du fascicule du brevet: **10.04.85**

(51) Int. Cl.[4]: **C 07 C 153/09**

(21) Numéro de dépôt: **81401928.7**

(22) Date de dépôt: **04.12.81**

## (54) Procédé de fabrication de thiochloroformiates.

(30) Priorité: **05.12.80 FR 8025819**

(43) Date de publication de la demande: **16.06.82 Bulletin 82/24**

(45) Mention de la délivrance du brevet: **10.04.85 Bulletin 85/15**

(84) Etats contractants désignés: **AT BE CH DE FR GB IT LI NL**

(56) Documents cités:
**EP - A - 0 024 683**
**US - A - 3 277 143**
**US - A - 3 299 114**

(73) Titulaire: **SOCIETE NATIONALE DES POUDRES ET EXPLOSIFS, 12, quai Henri IV, F-75181 Paris Cedex 04 (FR)**

(72) Inventeur: **Deweerdt, Julien, 5 Résidence de l'Ile Chemin de la Loge, F-31400 Toulouse (FR)**
Inventeur: **Sala, Jacques, Chemin de Jordanis Pinsaguel, F-31120 Portet sur Garonne (FR)**
Inventeur: **Souyri, Denis, 15, rue George Sand, F-31400 Toulouse (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 053 981 B1

ACTORUM AG

## Description

L'invention se rapporte à la fabrication des thiochloroformiates.

Il est connu de longue date qu'on peut obtenir des thiochloroformiates par réaction du phosgène sur un mercaptan. Ce procédé, qui fait appel à des matières premières très accessibles, présente cependant de graves inconvénients. L'un de ces inconvénients est la durée extrêmement longue de la réaction: il est fréquent que plusieurs jours soient nécessaires avant que soit atteint un degré acceptable d'avancement de la réaction. Un autre inconvénient est la formation de sous-produits en quantités non négligeables: il s'agit essentiellement de thiocarbonates symétriques et de disulfures qui en dérivent.

Deux solutions ont été proposée pour pallier notamment ces inconvénients. La plus ancienne de ces solutions consiste à utiliser du charbon actif comme catalyseur de la réaction. Cette solution est par exemple décrite dans le brevet français 1 298 704. Elle permet en effet de réduire notablement la durée de la réaction mais ne se montre pas entièrement satisfaisante quant à la réduction de la formation de sous-produits, du fait de l'existence de points chauds inévitables dans la charge de catalyseur. La nature de ce dernier est en outre la cause de difficultés de séparation du produit de la réaction et de colmatage des installations.

Des perfectionnements récents, décrits dans les brevets français 2 332 982 et 2 383 172, permettent, au prix d'une plus grande complexité de remédier à certains inconvénients du procédé ancien sans toutefois dispenser de l'usage du charbon actif.

La seconde solution a été décrite dans le brevet américain 3 227 143 et consiste en l'utilisation d'amides organiques, dans une proportion variable, comme catalyseurs. Dans ce cas, les durées de réaction sont réduites du même ordre de grandeur qu'avec les charbons actifs, mais il se forme au bout de quelques cycles des boues nuisant beaucoup à la bonne marche de l'installation, de sorte que ce procédé ne peut être valablement employé à l'échelle industrielle.

Il existe donc un besoin ancien non satisfait de disposer de catalyseurs efficaces mais ne présentant pas les inconvénients précédemment exposés.

La demanderesse a maintenant trouvé un procédé de fabrication de thiochloroformiates mettant en œuvre de tels catalyseurs.

Le procédé selon l'invention est un procédé de fabrication de thiochloroformiates par action du phosgène sur un mercaptan, en présence d'un catalyseur caractérisé en ce qu'on utilise comme catalyseur une urée substituée ou le produit de réaction d'une telle urée sur le phosgène.

Les urées convenant bien dans le cadre de l'invention sont notamment celles de formule générale:

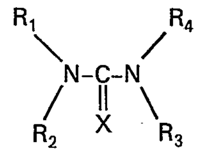

(1)

dans laquelle X = O ou S et dans laquelle $R_1$, $R_2$, $R_3$ et $R_4$ sont des radicaux alkyles linéaires ou ramifiés de $C_1$ à $C_{18}$, de préférence de $C_1$ à $C_8$, des radicaux aryles de $C_6$ à $C_9$, des radicaux alkaryles de $C_7$ à $C_{10}$, des aralkyles de $C_7$ à $C_{10}$, des radicaux alkényles de $C_2$ à $C_5$, ou des atomes d'hydrogène, les $R_1$, $R_2$, $R_3$ et $R_4$ pouvant être tous différents ou tous semblables, mais pas tous des atomes d'hydrogène, et pas tous des radicaux méthyle lorsque X = O ou bien encore $R_1$ et $R_2$ sont reliés entre eux pour former une chaîne polyméthylène comprenant de 4 à 8 chainons, $R_3$ et $R_4$ ayant une des valeurs précitées ou formant également entre eux une chaine polyméthylène comprenant de 4 à 8 chainons.

Selon une variante particulièrement préférée de l'invention, au moins deux des radicaux $R_1$, $R_2$, $R_3$ ou $R_4$ sont des groupes alkyles linéaires ou ramifiés de $C_1$ à $C_8$.

Comme urées particulièrement préférées on peut citer le tétraéthyl urée, la tétrapropylurée, la tétrabutylurée, la tétraoctylurée, la tétraphénylurée, la N,N,N'-tributyl urée, la N-N'-dibutyl urée, la N,N-diphényl urée, la N,N-dibenzyl urée, la tétra (p-tolyl) urée, la N,N'-di (p-tolyl) urée, la N,N'-diméthyl urée, la N',N'-diéthyl urée, la N,N-diéthyl N',N'-dipropyl urée, la N,N'-diisopropyl urée, la N,N-diméthyl N'-butyl urée, la N,N'-diisobutyl urée, la N-propyl N'-t butyl urée, la N,N' diisohexylurée, la N,N'-ditétradécylurée, la tétraméthylthiourée, la tétraéthylthiourée, la tétrabutylthiourée, la N-propyl N' isopropyl urée, la N,N-diméthyl N'-butyl urée, la tétraphénylthiourée, la dihexaméthylèneurée, ainsi que les produits de réaction de ces urées sur le phosgène.

La proportion de catalyseur utilisable conformément à la présente invention est en général compris entre 0,1 et 10% et de préférence entre 0,5 et 5% en moles par rapport au mercaptan. Ce taux est choisi faible lorsque la présence du catalyseur dans le produit final n'apparait pas comme gênante, par exemple entre 0,5 et 2% en moles. Il peut être choisi plus élevé si la réaction est effectuée dans un solvant. Par ailleurs, un facteur de sélection du catalyseur et de sa concentration est encore la facilité avec laquelle on peut le séparer du mélange réactionnel, par exemple par distillation: dans ce cas, on choisit un catalyseur conforme à l'invention dont le point d'ébulition s'écarte notablement de celui du thiochloroformiate formé.

Le procédé selon l'invention peut être mis en œuvre de manière discontinue ou continue. La réaction peut être pratiquée en l'absence de solvant ou bien en présence d'un solvant inerte vis à vis du phosgène qui peut être, par exemple, un solvant aromatique tel que le chlorobenzène, le toluène, un xylène ou un solvant aliphatique chloré tel que le dichlorométhane ou bien encore le produit de la réaction luimême, notamment dans le cas également envisagé selon l'invention d'une réaction effectuée dans un réacteur en boucle. De tels solvants sont choisis en fonction de leur aptitude à dissoudre à la fois le mercaptan et le phosgène, la partie de ce dernier dissoute dans le milieu étant apparue comme celle participant le plus à la réaction.

Les mercaptans intéressés par la présente invention sont ceux de formule générale R S H dans laquelle R est un groupe alkyle linéaire ou ramifié de préférence de $C_1$ à $C_{10}$, un groupe alkényle de $C_2$ à $C_8$ tel qu'un groupe allyle, un groupe cycloalkyle ou cycloalkyl-méthyle de $C_5$ à $C_{12}$, un groupe aromatique de $C_6$ à $C_{10}$ tel qu'un groupe phényle, chlorophényle, bromophényle, ou benzyle ou encore un groupe alkyle substitué n'empêchant pas la réaction du phosgène sur le mercaptan, tel que le chloro-2 propyle. Le procédé selon l'invention permet d'accéder, d'une manière générale, aux thiochloroformiates conduisant aux thiocarbamates rentrant dans grand nombre de compositions herbicides.

Conformément au procédé selon l'invention, la réaction est effectuée à une température voisine de la température ambiante, entre −10° et +70°C, de préférence entre 5 et 50°C.

D'une manière générale, il est préférable d'éviter d'effectuer la réaction en présence d'un trop grand excès de mercaptan, du fait de la grande efficacité du catalyseur: en effet, en présence d'un tel excès, il pourrait avoir réaction du thiochloroformiate déjà formé sur le mercaptan n'ayant pas réagi et formation de thiocarbonates et de disulfures. En conséquence, on préfère former une charge initiale de thiochloroformiate dans laquelle on introduit, de préférence simultanément, le phosgène et le mercaptan. Un excès de phosgène, qui se dissout bien dans le thiochloroformiate, n'est pas un inconvénient.

Au contraire, on favorise avantageusement cet excès et on maintient cet avantage en introduisant le phosgène sous forme finement divisée, par exemple à l'aide d'un injecteur couplé à des moyens de refroidissement du milieu réactionnel. Dans un procédé continu notamment, le stade d'équilibre étant atteint, un excès de phosgène de 0,5 à 10% en moles par rapport au mercaptan donne satisfaction.

S'il n'est pas nécessaire d'opérer sous atmosphère inerte, il est par contre avantageux d'opérer à une pression supérieure à la normale allant de 1 à 10 atmosphères, par exemple. Toutefois les catalyseurs selon l'invention conduisant déjà à des résultats très satisfaisants à la pression atmosphérique, il n'est généralement pas nécessaire de recourir à cet expédient qui engendre quelques contraintes d'appareillage.

La réaction proprement dite étant effectuée de préférence dans un milieu agité, ce qui se réalise à l'aide d'un réacteur agité ou bien d'un injecteur, par exemple dans un réacteur fonctionnant en boucle et permettant une fabrication en continu, il est rarement nécessaire de prévoir, de manière connue par l'homme du métier, un mûrissement du milieu réactionnel dans un réacteur auxiliaire muni ou non de moyens permettant d'éviter le rétro-mélangeage.

Le temps de réaction est, suivant les conditions opératoires, d'environ 1 à 10 heures. Le rendement obtenu au terme de la réaction est généralement supérieur à 90% et de l'ordre de 97 à 99%.

La pureté du produit obtenu est supérieure à 97% en poids et généralement de l'ordre de 98%. Ainsi, pour la plupart des applications des thiochloroformiates, est-il possible de laisser le catalyseur dans le produit final sans diminuer la réactivité du produit et tout en conservant une pureté satisfaisante.

Compte tenu de la faiblesse des progrès enregistrés dans le domaine de la fabrication des thiochloroformiates depuis de décennies, on appréciera d'autant plus les avantages procurés par la présente invention: adoucissement des conditions opératoires (basses températures, pression normale), raccourcissement des temps de réaction et amélioration des rendements et de la pureté, non formation de boues, ni constatation de perte d'activité du catalyseur, après plusieurs recyclages.

Ces avantages seront mieux appréciés et l'invention mieux comprise à l'aide des exemples non limitatifs suivants.

Exemple 1

Dans un réacteur de 1 litre muni d'un dispositif d'agitation, d'un dispositif d'introduction de phosgène, d'un dispositif de refroidissement et de condenseurs efficaces, on a placé 103 g de thiochloroformiate d'octyle et 42 g de tétrabutylurée.

On a d'abord introduit dans le réacteur 30 g de phosgène puis, simultanément, sous agitation, 330 g phosgène et 438 g d'octyl mercaptan, en 2 heures, tout en maintenant une température de l'ordre de 18°C. La quantité de catalyseur par rapport au thiol s'élevait donc à 5% en moles.

A la fin de la coulée, on a respecté un palier d'une heure à la même température de 18°C après quoi on a dégazé le produit sous l'action combinée du vide et d'un barbotage d'azote.

Le produit obtenu montrait une température d'ébullition de 105°C (sous 2 mm d'Hg) et une pureté de 99%, en chromatographie en phase gazeuse.

On a obtenu 666 g de thiochloroformiate d'octyle, soit un rendement de 90%.

Exemple 2

Dans un réacteur de 10 litres muni d'une agitation et de systèmes de condensation efficaces, d'une prise de température et d'un dispositif d'introduction du phosgène, on a placé 1 kg de thiochloroformiate d'octyle provenant d'une fabrication précédente.

On a ensuite introduit dans le réacteur agité, successivement, 41 g de tétrabutylurée (soit 0,144 mole) et 0,9 kg de phosgène. Ensuite, on a introduit dans le réacteur, toujours agité, simultanément 3 kg d'octyl mercaptan (20,5 moles) et 1,35 kg de phosgène en 2 heures, la température étant maintenue aux environs de 30°C. On a observé, dès l'introduction terminée, un palier d'une heure à 30°C après quoi on a dégazé le mélange réactionnel comme à l'exemple 1.

On a obtenu 5,3 kg de mélange réactionnel brut contenant:

- 98,2% de thiochloroformiate d'octyle,
- 0,8% de tétrabutylurée,
- 0,6% de disulfure d'octyle,
- 0,4% de dithiocarbonate d'octyle.

Le rendement de l'opération est de l'ordre de 98%.

Exemple 3

Dans un réacteur agité de 20 litres, on a mis en œuvre le procédé décrit à l'exemple 2, mais sans pied de cuve, avec 277 g de tétrabutylurée, et 1 kg de phosgène (soit 10,1 moles) introduits tout d'abord dans le réacteur.

On a ensuite introduit 6,055 kg d'éthyl mercaptan (soit 97,6 moles) et 9,6 kg de phosgène (soit 97 moles) en maintenant une température de 20°C.

A la fin de l'introduction, on a observé un palier de 3 heures à cette température. On a dégazé puis distillé le produit obtenu qui présentait un point d'ébullition de 50°C sous 31 mm de mercure. On a recueilli 10,9 kg de thiochloroformiate d'éthyle, soit un rendement de 90%.

Exemple 4

Dans un réacteur de 10 litres muni agitateur, d'un condenseur, d'une prise de température, d'une ampoule de coulée, d'un dispositif d'introduction de phosgène et muni d'une double enveloppe de refroidissement, on a placé un pied de cuve de 500 g de thiochloroformiate d'octyle.

A ce pied de cuve on a rajouté 16 g (0,137 moles) de tétraméthylurée puis 400 g de phosgène (4,04 moles).

Ensuite, dans le mélange obtenu, on a introduit simultanément 2 kg d'octyl mercaptan (13,69 moles) et 1,7 kg de phosgène (17,1 mole) en 2 h 30, à une température de 20°C.

Après ces introductions on a laissé sous agitation 5 h 30 à 20°C. On a dégazé alors le milieu sous vide et on a finalement obtenu 3,240 kg de thiochloroformiate d'octyle titrant 98,4% (analyse par chromatographie en phase gazeuse), soit un rendement de 95,9%.

Exemple 5

Dans un réacteur d'un litre, refroidi à 0°C, on a introduit 70 g de phosgène liquide et 21,2 g (0,1 mole) de N,N'-diphénylurée. On a introduit ensuite simultanément 292 g (2 moles) d'octyl mercaptan et 180 g (1,82 mole) de phosgène en 1 h 15. Lors de la coulée des réactifs, la température s'est accrue et a atteint 18°C.

A la fin de l'introduction des réactifs, on a laissé sous agitation pendant 5 h 30 puis on a dégazé sous vide. Le produit brut obtenu a été filtré et on a recueilli 375 g d'un produit contenant 90,2% de thiochloroformiate d'octyle.

Ce produit a distillé à 70°C sous un vide de 0,15 mm de mercure.

Exemple 6

On a utilisé le mode opératoire de l'exemple 5, avec 146 g (1 mole) d'octyl mercaptan et 90 g de phosgène. Le pied de cuve initial contenait 35 g de phosgène liquide et 5,8 g (0,05 mole) de N,N'-diéthylurée.

On a obtenu 200 g de produit brut dégazé contenant 92% de thiochloroformiate d'octyle.

On a distillé ce produit sous 0,1 torr à 66°C et on a obtenu 166,9 g de thiochloroformiate d'octyle très pur.

Exemple 7

Dans un réacteur d'un litre on a placé 438,9 g (3 moles) d'octyl mercaptan et 24,6 g diisopropylthiourée qui n'est pas soluble dans le thiol.

On a refroidi à 5°C et on a alimenté en phosgène. On a opéré avec un léger reflux sur le condenseur à −25°C. La température de phosgénation était de 18 à 21°C et le débit de phosgène de 65 l/h. On a introduit au total 605 g de phosgène (6 moles) en 2 heures. L'excès de phosgène était de 100%. On n'a pas observé de gros reflux de phosgène en fin de réaction mais la disparition du précipité de thiourée au bout d'1 h 30 de phosgénation.

On a suivi la réaction par chromatographie en phase gazeuse: la teneur en thiochloroformiate était respectivement de 51-75,6-80,25 et 96% en poids au bout de 1-1,5-2,5 et 5,15 heures.

**Revendications**

1. Procédé de fabrication de thiochloroformiates par action du phosgène sur un mercaptan en présence d'un catalyseur, caractérisé en ce qu'on utilise comme catalyseur une urée substituée de formule générale:

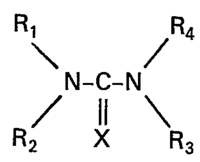

dans laquelle X = O ou S et dans laquelle $R_1$, $R_2$, $R_3$ et $R_4$ sont des radicaux alkyles linéaires ou ramifiés de $C_1$ à $C_{18}$, de préférence de $C_1$ à $C_8$, des radicaux aryles de $C_6$ à $C_9$, des radicaux alkaryles de $C_7$ à $C_{10}$, des aralkyles de $C_7$ à $C_{10}$, des radicaux alkényles de $C_2$ à $C_5$, ou des atomes d'hydrogène, les $R_1$, $R_2$, $R_3$ et $R_4$ pouvant être tous différents ou tous semblables, mais pas tous des atomes d'hydrogène, et pas tous des radicaux méthyle lorsque X = O, ou bien encore $R_1$ et $R_2$ sont reliés entre eux pour former une chaîne polyméthylène comprenant de 4 à 8 chainons, $R_3$ et $R_4$ ayant une des valeurs précitées ou formant également entre eux une chaine polyméthylène comprenant de 4 à 8 chainons; ou le produit de réaction d'une

telle urée sur le phosgène, et en ce que le taux de catalyseur est compris entre 0,5 et 5% en moles par rapport au mercaptan.

2. Procédé selon la revendication 1, caractérisé en ce que l'urée est la tétrabutylurée.

3. Procédé selon l'une quelconque des revendications précédentes, caractérisé en ce que la réaction est effectuée en présence d'un solvant inerte vis à vis du phosgène.

4. Procédé selon la revendication 3, caractérisé en ce que le solvant est le produit de la réaction lui-même.

5. Procédé selon l'une quelconque des revendications précédentes, caractérisé en ce que la réaction est effectuée à une température voisine de la température ambiante, entre −10° et +70°C, de préférence entre 5 et 50°C.

6. Procédé selon l'une quelconque des revendications précédentes, caractérisé en ce que le procédé est continu.

7. Procédé selon la revendication 6, caractérisé en ce qu'on utilise, le stade d'équilibre étant atteint, un excès de phosgène de 0,5 à 10% en moles par rapport au mercaptan.

**Patentansprüche**

1. Verfahren zur Herstellung von Thiochlorameisensäureestern durch Umsetzen von Phosgen und einem Merkaptan in Gegenwart eines Katalysators, dadurch gekennzeichnet, dass man als Katalysator einen substituierten Harnstoff der allgemeinen Formel:

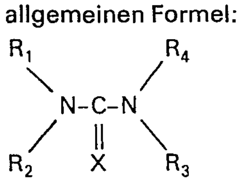

in der X = O oder S ist und $R_1$, $R_2$, $R_3$ und $R_4$ geradkettige oder verzweigte $C_{1-18}$-Alkylreste, vorzugsweise $C_{1-8}$-Alkylreste, $C_{6-9}$-Arylreste, $C_{7-10}$-Alkylarylreste, $C_{7-10}$-Arylalkylreste, $C_{2-5}$-Alkenylreste oder Wasserstoffatome sind, wobei $R_1$, $R_2$, $R_3$ und $R_4$ alle gleich oder verschieden sein können, aber nicht alle Wasserstoffatome, und nicht alle Methylreste sein können, wenn X = O, oder $R_1$ und $R_2$ miteinander verbunden sind und eine 4- bis 8gliedrige Polymethylenkette bilden, $R_3$ und $R_4$ eine der vorstehenden Bedeutung haben oder auch eine 4- bis 8gliedrige Polymethylenkette bilden, oder das Reaktionsprodukt eines solchen Harnstoffs mit dem Phosgen verwendet und die Menge des Katalysators von 0,5 bis 5 Mol-%, bezogen auf das Merkaptan, beträgt.

2. Verfahren nach Anspruch 1, dadurch gekennzeichnet, dass der Harnstoff Tetrabutylharnstoff ist.

3. Verfahren nach einem der vorstehenden Ansprüche, dadurch gekennzeichnet, dass man die Reaktion in Gegenwart eines gegenüber dem Phosgen inerten Lösungsmittel durchführt.

4. Verfahren nach Anspruch 3, dadurch gekennzeichnet, dass das Lösungsmittel das Reaktionsprodukt selbst ist.

5. Verfahren nach einem der vorstehenden Ansprüche, dadurch gekennzeichnet, dass man die Reaktion bei einer der Umgebungstemperatur nahen Temperatur, von −10° bis +70°C, vorzugsweise von 5 bis 50°C, durchführt.

6. Verfahren nach einem der vorstehenden Ansprüche, dadurch gekennzeichnet, dass es kontinuierlich ist.

7. Verfahren nach Anspruch 6, dadurch gekennzeichnet, dass man bei Erreichen des Gleichgewichtes einen Überschuss an Phosgen von 0,5 bis 10 Mol-%, bezogen auf das Merkaptan, verwendet.

**Claims**

1. Process for the manufacture of thiochloroformates by reacting phosgene with a mercaptan in the presence of a catalyst, characterised in that there is used as catalyst a substituted urea of the general formula:

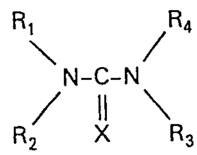

in which X = O or S and in which $R_1$, $R_2$, $R_3$ and $R_4$ are linear or branched $C_1$ to $C_{18}$, preferably $C_1$ to $C_8$, alkyl radicals, $C_6$ to $C_9$ aryl radicals, $C_7$ to $C_{10}$ alkaryl radicals, $C_7$ to $C_{10}$ aralkyl radicals, $C_2$ to $C_5$ alkenyl radicals or hydrogen atoms, it being possible for all of $R_1$, $R_2$, $R_3$ and $R_4$ to be different or similar, but not all hydrogen atoms and not all methyl radicals when X = O, or alternatively $R_1$ and $R_2$ are joined to one another to form a polymethylene chain containing from 4 to 8 members, $R_3$ and $R_4$ having one of the meanings mentioned above or also forming with one another a polymethylene chain containing from 4 to 8 members, or the reaction product of such a urea with phosgene, and in that the proportion of catalyst is between 0.5 and 5 mole % relative to the mercaptan.

2. Process according to Claim 1, characterised in that the urea is tetrabutylurea.

3. Process according to any one of the preceding claims, characterised in that the reaction is carried out in the presence of a solvent which is inert towards phosgene.

4. Process according to Claim 3, characterised in that the solvent is the reaction product itself.

5. Process according to any one of the preceding claims, characterised in that the reaction is carried out at a temperature close to ambient temperature, namely at between −10° and +70°C and preferably between 5 and 50°C.

6. Process according to any one of the preceding claims, characterised in that the process is continuous.

7. Process according to Claim 6, characterised in that, when the equilibrium state has been reached, a 0.5 to 10 mole % excess of phosgene is used, relative to the mercaptan.